# EUROPEAN PATENT SPECIFICATION

(11) **EP 1 663 077 B1**
(45) Date of publication and mention of the grant of the patent: **09.01.2008**
(21) Application number: 04769277.7
(22) Date of filing: 02.09.2004
(51) Int. Cl.: A61F 2/36

(54) **SET OF MOBILE NECKS FOR INSERTING INTO THE STEM OF A HIP PROSTHESIS**
BEWEGLICHES HALSSET ZUM EINSETZEN IN DEN SCHAFT EINER HÜFTPROTHESE
ENSEMBLE DE COLS AMOVIBLES A INTRODUIRE DANS LA TIGE D'UNE PROTHESE DE LA HANCHE

(30) Priority: 18.09.2003 CH 159903
(43) Date of publication of application: 07.06.2006
(73) Proprietor: Mariasal Investment N.V., Curaçao (AN)
(72) Inventor: RAGBIR, Sheila, Whim, Trinidad and Tobago (TT)
(74) Representative: Fiammenghi-Domenighetti, Delfina
(86) International application number: PCT/IB2004/002874
(87) International publication number: WO 2005/025460

(56) References cited:
- EP-A- 0 201 407
- EP-A- 0 797 964
- DE-A- 4 407 227
- DE-C- 3 600 804

## Description

The present invention relates to the technological sector of the component parts of a hip prosthesis.

As is known to those skilled in the art, these component parts consist of a stem, which is fitted into the cavity of the femur, and into this stem is inserted a "neck" (referred to as mobile in the sense that it does not form an integral whole with the said stem), fitted to the free end of which is a spherical head designed to mate with the cavity of the acetabulum.

Mobile necks of the type described above were devised to make it possible, as the need arose, by changing the type of neck used, to create a prosthesis that is anatomically and ergonomically suitable for the skeleton of the patient being fitted with a hip prosthesis.

Mobile necks are therefore produced in sets so that their various dimensions can be used to cover all possible cases as disclosed in EP 201 407. In these sets each neck has a different length and inclination, in such a way that, depending on the physical structure of the patient, the prosthesis is suitable for that structure. In an intervention to fit a hip prosthesis, two important dimensions must be observed: the so-called off-set, which is the distance between the axis of the stem and the centre of rotation of the head, and the height, meaning the distance between a plane perpendicular to the axis of the stem where it connects with the neck, and the abovementioned centre of rotation of the head.

As a consequence, with the sets of mobile necks currently existing on the market, since it is only possible to modify the length or the inclination, the ideal value is obtained for usually only one of these two dimensions, that is either the offset or the height. The reason for this, of course, is that the desired offset can be obtained by for example reducing the length of the mobile neck, but this would simultaneously reduce the height in a way which might not always be acceptable if the intervention is to be successful.

On the other hand there is no question of supplying "made-to-measure" mobile necks in the sense of having the right length and the right inclination relative to the stem, because in the first place the exact measurements can only be found out during the intervention, and in any case the costs would be prohibitive, precluding many people from the chance of undergoing an intervention to fit a hip prosthesis.

The inventor of the subject of the present application has, in order to avoid all of the drawbacks set out above, devised a set of necks to meet the requirements of all possible hip prosthesis cases. Thus, using in each case one of the necks taken from the said set, it is possible to have hip prosthesis components at ones disposal that are sufficiently adapted to the anatomical structure of the patient, in such a way as to avoid the kind of large imprecisions that can cause sometimes painful post-operative complications that would lead to the intervention being pronounced a failure.

The set of the invention, which makes it possible to obtain the functional advantages described above, is composed of a perfectly acceptable minimum number (15) of necks, all different from each other so as to cover with sufficient precision all possible situations, and can therefore be produced without significantly increasing costs compared with the cost of some present-day sets composed of around ten pieces.

In the first place, as will be seen more clearly below, the 15 mobile necks mentioned above can also cover all the diverse situations of retroversion and anteversion with a single value of off-set in ante/retroversion. In a set of mobile necks according to the invention, the necks are produced with different inclinations and lengths in such a way that their free ends, when inserted in a stem, terminate at nine mutually equidistant points arranged in three parallel rows perpendicular to the axis V-V of the stem 2, likewise equidistant, in such a way that the lines joining the outermost points delineate a square, the length of whose sides is 15 mm, the diagonal of this square coinciding with the axis of a mobile neck inclined at 45°, that is set at a neutral inclination (neither varus nor valgus) with respect to a plane perpendicular to the axis of the abovementioned stem.

The subject of the present invention is therefore a set of mobile necks as described in the accompanying Claim 1.

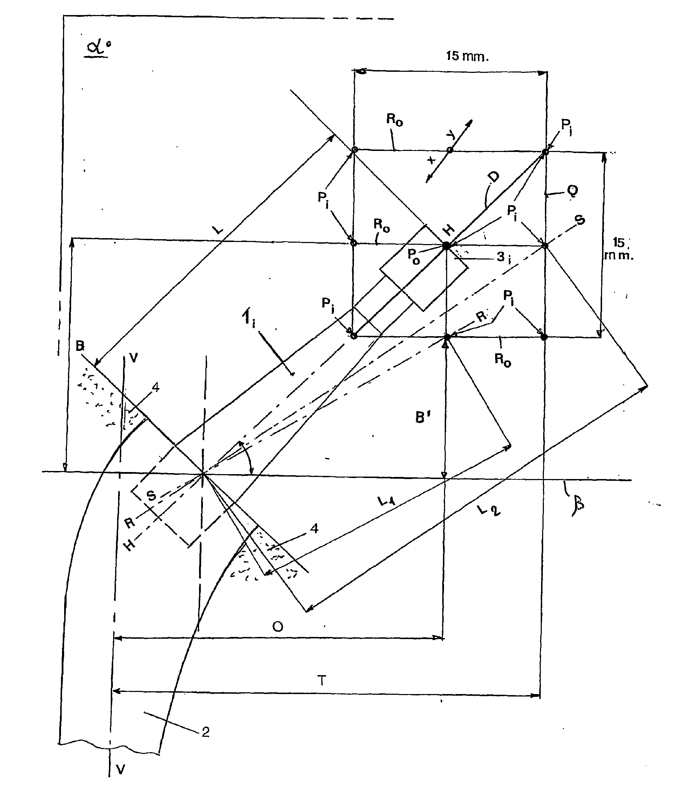

A more detailed description will now be given of an illustrative embodiment of a set according to the invention reference also being made to the accompanying figure, which is an enlarged diagram illustrating the various possible geometrical characteristics of each neck forming the said set.

Referring to the figure, this shows that nine mutually equidistant points Pᵢ are arranged in threes in three parallel rows Rₒ perpendicular to the axis V-V of the stem 2, which are likewise equidistant from each other.

The figure also shows part of the stem 2, inserted into a femur 4, into which there is inserted by known methods one end of a mobile neck 1i belonging to the set of the invention, the free end 3i of which is shaped to allow it to be housed in a spherical head (not shown).

The said free end 3i terminates along the axis H-H of the mobile neck at one of the points Pᵢ, marked Pₒ in the figure, and therefore has a length L and an inclination of 45° corresponding to a neutral neck, that is neither varus nor valgus.

This neck 1i (which in the figure terminates as stated at the point Pₒ) gives rise to one particular off-set measurement O and one particular height measurement B. If the anatomical structure of the patient requires the same off-set but a height B' smaller than B, the surgeon simply chooses from the set of mobile necks a neck of length L₁, of which only the axis R-R is shown with a smaller inclination, and the desired result is obtained.

Similarly, if an off-set 0' (that is a distance from the axis V-V of the stem 2) greater than O is required, but the same height B, the surgeon simply selects a mobile neck from among the set of the invention (of which again only the axis S-S is depicted) of length L₂, which generates an off-set T greater than O, but which keeps the height B.

The fact that the number of points Pᵢ corresponding to an equivalent number of mobile necks 1i is limited to nine could leave areas in the square Q where none of the mobile necks of the set terminates, but this is overcome by varying in a known manner the type of head fitted, i.e. by varying the depth of the cavity formed inside it to take the end 3i of a mobile neck 1i: modifying this depth has the effect (see arrows x, y) of "lengthening" or "shortening" the neck, moving the centre of rotation of the head in the two directions, and thus filling the areas described earlier.

It should be pointed out that, on the mobile neck 1i illustrated in full, it is intended that the terminal point of its axis H-H lying on the end 3i should coincide with the centre of rotation of the head that is fitted to this end 3i.

As regards how the mobile necks are inclined in anteversion and retroversion, that is with respect to the frontal plane α of a person containing the centres of the articulations of the two legs of said patient, it is sufficient, given the symmetry of the prostheses of limbs, to give the desired different inclinations to a limited number of mobile necks within the set of the invention, with the result already described earlier that as few as 15 mobile necks 1i, all different from each other, as stated, can create a set complete enough to be used in practically all cases normally treated. For this purpose the inventor recommends that the distance between the points Pᵢ be approximately 7.5 mm in both directions, so that the square Q enclosing them has a side of length 15 mm.

Another note concerns the definition of length (L, L₁, L₂) of a mobile neck: obviously, this length is the actual axial length of the mobile neck when the inclination of the latter is not corrected in either anteversion or retroversion. In the other cases this length corresponds to the projection onto the abovementioned plane α of the actual length of the mobile neck.

## Claims

1. Set of mobile necks (1i) for inserting into a stem (2) of a hip prosthesis, where the said mobile necks (1i) are produced with different inclinations and lengths and comprise a free end (3i) and an axis (H-H), and the stem (2) comprises an axis (V-V), **characterized in that** the free ends (3i), when inserted in the said stem (2), terminate at nine mutually equidistant points (Pᵢ) arranged in three parallel rows (Rₒ) perpendicular to the axis (V-V) of the stem (2) in such a way that the lines joining the outermost points delineate a square (Q), the length of whose sides is approximately 15 mm, the diagonal (D) of this square coinciding with the axis (H-H) of a mobile neck (1) set at a neutral inclination, that is neither varus nor valgus, with respect to a plane (β) essentially perpendicular to the axis of the abovementioned stem (2).

2. Set according to Claim 1, in which the axes of at least some of the said mobile necks (1i) are also inclined in such a way as to form the said square (Q) of nine points with a desired displacement of these points in anteversion or retroversion with respect to a frontal plane (α) of a patient containing the centres of the articulations of the two legs of said patient.

## Patentansprüche

1. Satz aus mobilen Hälsen (1i) zum Einsetzen in einen Schaft (2) einer Hüftprothese, wobei die mobilen Hälse (1i) mit verschiedenen Neigungen und Längen hergestellt werden und ein freies Ende (3i) und eine Achse (H-H) umfassen, und wobei der Schaft (2) eine Achse (V-V) umfasst, **dadurch gekennzeichnet, dass** die freien Enden (3i), wenn sie in den Schaft (2) eingesetzt sind, an neun gleichmäßig voneinander beabstandeten Punkten (Pᵢ) abschließen, die in drei parallelen Reihen (Rₒ), die senkrecht zu der Achse (V-V) des Schaftes (2) verlaufen, dergestalt angeordnet sind, dass die Linien, welche die äußersten Punkte verbinden, ein Quadrat (Q) beschreiben, wobei die Länge der Seiten dieses Quadrats etwa 15 mm beträgt, wobei die Diagonale (D) dieses Quadrats mit der Achse (H-H) eines mobilen Halses (1) übereinstimmt, der auf eine neutrale Neigung, die weder nach innen noch nach außen gekrümmt ist, mit Bezug auf eine Ebene (β) eingestellt ist, die im Wesentlichen senkrecht zu der Achse des Schaftes (2) verläuft.

2. Satz nach Anspruch 1, wobei die Achsen mindestens einiger dieser mobilen Hälse (1i) ebenfalls in einer solchen Weise geneigt sind, dass das Quadrat (Q) aus neun Punkten mit einer gewünschten Verschiebung dieser Punkte in Vorwärtsneigung oder Rückwärtsneigung mit Bezug auf eine frontale Ebene (α) eines Patienten, welche die Mittelpunkte der Gelenke der beiden Beine des Patienten enthält, gebildet wird.

## Revendications

1. Ensemble de cols mobiles (1i) à introduire dans une tige (2) d'une prothèse de hanche, dans lequel lesdits cols mobiles (1i) sont réalisés selon différentes inclinaisons et avec différentes longueurs et comprennent une extrémité libre (3i) et un axe (H-H), et la tige (2) comprend un axe (V-V), **caractérisé en ce que** les extrémités libres (3i), lorsqu'elles sont insérées dans ladite tige (2), aboutissent en neuf points mutuellement équidistants (Pᵢ) disposés en trois rangées parallèles (R₀) perpendiculaires à l'axe (V-V) de la tige (2), de sorte que les lignes reliant les points situés le plus à l'extérieur délimitent un carré (Q), dont les côtés ont une longueur d'environ 15 mm, la diagonale (D) de ce carré coïncidant avec l'axe (H-H) d'un col mobile (1) disposé avec une inclinaison neutre, qui n'est ni varus ni valgus, par rapport à un plan (β) pratiquement perpendiculaire à l'axe de la tige susmentionnée (2).

2. Ensemble selon la revendication 1, dans lequel les axes d'au moins certains desdits cols mobiles (1i) sont également inclinés de façon à former ledit carré (Q) de neuf points avec un déplacement souhaité de ces points, en antéversion ou rétroversion vis-à-vis d'un plan frontal (α) d'un patient contenant les centres des articulations des deux jambes dudit patient.
